# EUROPÄISCHE PATENTSCHRIFT

(11) **EP 2 358 652 B1**
(45) Veröffentlichungstag und Bekanntmachung des Hinweises auf die Patenterteilung: **19.08.2015**
(21) Anmeldenummer: 09796942.2
(22) Anmeldetag: 25.11.2009
(51) Int. Cl.: C07C 1/20, C07C 11/04, C07C 11/06

(54) **VERFAHREN ZUM HERSTELLEN EINES C3H6 UND C2H4 ENTHALTENDEN PRODUKTS**
METHOD FOR PRODUCING A PRODUCT CONTAINING C3H6 AND C2H4
PROCÉDÉ POUR PRODUIRE UN PRODUIT CONTENANT C3H6 ET C2H4

(30) Priorität: 11.12.2008 DE 102008061300
(43) Veröffentlichungstag der Anmeldung: 24.08.2011
(73) Patentinhaber: Air Liquide Global E&C Solutions Germany GmbH, 60439 Frankfurt am Main (DE)
(72) Erfinder: BUCHOLD, Henning, 63452 Hanau (DE); KÖMPEL, Harald, 63263 Neu-Isenburg (DE); POHL, Sven, 60388 Frankfurt a.M. (DE); ROTHAEMEL, Martin, 60437 Frankfurt am Main (DE); WAGNER, Ulrich, 06406 Bernburg (DE)
(86) Internationale Anmeldenummer: PCT/EP2009/008395
(87) Internationale Veröffentlichungsnummer: WO 2010/066339

(56) Entgegenhaltungen:
- WO-A1-2009/065870
- DD-A3- 257 740
- US-A- 4 542 252

## Beschreibung

Die Erfindung betrifft ein Verfahren zum Herstellen eines C₃H₆ und C₂H₄ enthaltenden Produkts durch zeitgleiches Umsetzen von MeOH und EtOH in einem mehrere Reaktionsstufen enthaltenden adiabaten, sequentiell betriebenen Reaktor, wobei jede Reaktionsstufe mit einem Festbett aus formselektivem Katalysator belegt ist, indem ein gasförmiger MeOH, DME, H₂O und ggf. einen oder mehrere C₂-, C₄-, C₅-, C₆-, C₇-, C₈-Olefine und -Paraffine enthaltender Einsatzstrom mit Temperaturen von 300 bis 600° C bei Drücken von 0,1 bis 20 bar[a] wenigstens der ersten Reaktionsstufe des Reaktors aufgegeben wird.

In der petrochemischen Industrie gehört das Herstellen binärer Copolymere mit einem definierten Anteil an C₃H₆ und C₂H₄, typischerweise 8 % C₂H₄ und 92 % C₃H₆, zu den am stärksten wachsenden Produktbereichen. Dementsprechend sind Verfahren zur gezielten Herstellung dieser Gemische aus C₂H₄ und C₃H₆ von besonderem Interesse. Ferner ist man bestrebt, als Ausgangsmaterial für die Herstellung von Olefinen anstelle von Erdöl mittel- und langfristig andere Rohstoffe einzusetzen.

Aus der DE 197 23 363 A1 ist ein Verfahren zum Erzeugen von C₂- und C₄-Olefinen durch Umsetzen eines MeOH und/oder DME und H₂O enthaltenden dampfförmigen Reaktionsgemisches in einem ersten Reaktor an einem formselektiven Katalysator mit Temperaturen von 280 bis 570° C bei Drücken von 0,1 bis 1 bar[a] bekannt, wobei aus dem ersten Reaktor ein C₂- bis C₄-Olefine und Benzinkohlenwasserstoffe (C₅₊) enthaltendes Produktgemisch abgezogen und ein aus den abgetrennten Benzinkohlenwasserstoffen reicher C₅₊-Strom verdampft wird, mit Wasserdampf gemischt wird, das Gewichtsverhältnis H₂O zu Kohlenwasserstoffe auf 0,5 :1 bis 3 : 1 eingestellt und das erzeugte Gemisch mit Temperatur von 380 bis 700° C in einen zweiten einen formselektiven Katalysator enthaltenden Reaktor geleitet und ein Produktgemisch abgezogen wird, dessen summierter Gehalt an C₃H₆ und Butenisomeren mindestens 50 Gew. % der Olefin-Bestandteile des dem zweiten Reaktor zugeführten Einsatzgemisches beträgt.

Die DE 100 271 59 A1 beschreibt das sog. MTP^{®}-Verfahren zum Herstellen von C₃H₆ aus MeOH, bei dem dampfförmiges MeOH an einem ersten Katalysator zu einem DME enthaltenden ersten dampfförmigen Gemisch umgesetzt und an einem formselektiven in mindestens zwei in Serie geschalteten adiabaten Schachtreaktoren angeordneten Zeolith-Katalysator ein C₃H₆ enthaltendes Produktgemisch erzeugt wird, wobei ein erster Teilstrom des DME enthaltenden ersten Dampfgemisches zusammen mit H₂O-Dampf in den ersten Schachtreaktor geführt, aus dem ein erstes Zwischenproduktgemisch abgezogen und dem zweiten Schachtreaktor aufgegeben wird. Dem zweiten Schachtreaktor wird ein zweiter Teilstrom des DME enthaltenden ersten Dampfgemisches zugeführt und aus dem letzten der in Serie geschalteten Schachtreaktoren ein Produktgemisch abgezogen und von dem eine C₃H₆-reiche Fraktion abgetrennt wird. Der verbleibende teilweise gasförmige, C₂H₄ und C₄₊-Kohlenwasserstoffe enthaltende Rest wird in einen der Schachtreaktoren rückgeführt.

Gegenstand der US 2005/0107651 A1 ist ein Verfahren zum Herstellen eines MeOH und EtOH enthaltenden Gemisches und das Umwandeln von MeOH und EtOH zu leichten Olefinen, wobei MeOH und EtOH mit einem Gewichtsverhältnis von 1 bis 99 % einer Reaktionszone zugeführt werden, in der MeOH und EtOH an einem Molekularsieb-Katalysator zu leichten Olefinen umgesetzt werden. Bei einem ähnlichen aus der WO 2005/051872 A1 bekannten Verfahren beträgt das Verhältnis von MeOH zu EtOH im Einsatzstrom auf 6 bis 10 eingestellt.

Die DE 102 33 975 A1 befasst sich mit der Herstellung von C₃H₆ aus MeOH, bei dem ein dampfförmiges Gemisch aus MeOH, DME und H₂O bei Betriebstemperaturen von 250 bis 460° C über mehrere sequentiell angeordnete jeweils mit einem formselektiven Zeolithkatalysator mit Pentasilstruktur ausgestattete Reaktionsstufen eines adiabaten Reaktors geleitet wird und zwischen den Reaktionsstufen eine Abkühlung des austretenden Reaktionsgemisches stattfindet. Nach der letzten Reaktionsstufe wird das abgekühlte Reaktionsgemisch in eine Gasphase und in eine überwiegend Wasser enthaltende Flüssigkeitsphase getrennt und die Gasphase nach einer Verdichtung in eine Kohlenwasserstoffe enthaltende Gasphase und in eine DME, MeOH und Wasser enthaltende Flüssigkeitsphase getrennt und danach C₃H₆ von der Gasphase entfernt. Bei einer vorteilhaften Weiterentwicklung dieses Verfahrens hinsichtlich einer verbesserten Wärmeführung wird gemäß DE102 006 026 103 A1 ein DME und MeOH sowie H₂O enthaltender Prozessstrom abgekühlt und in eine Flüssigkeits- und in eine Gasphase getrennt, die Gas- und Flüssigkeitsphase in mehrere Teilströme, deren Anzahl jeweils der Anzahl der zwischen den Reaktionsstufen bestehenden Zwischenräume entspricht, aufgeteilt und der einzelne Gasphasen-Teilstrom mit jeweils einem Flüssigphasen-Teilstrom einem Düsenrohr aufgegeben und die Flüssigphase mittels der Gasphase in den korrespondierenden Zwischenraum versprüht.

Die DD 257 740 A3 betrifft ein Verfahren zum Erzeugen von C₂- bis C₄-Olefinen durch Umsetzen von CO und H₂ enthaltenden Gasen zu einem Alkoholgemisch, das MeOH und höhere aliphatische Alkohole in einem Massenverhältnis von MeOH zu höheren Alkoholen von 0,6 bis 5,6 enthält, wobei die höheren aliphatischen Alkohole an zeolithischen Katalysatoren mit Pentasilstruktur bei Temperaturen von 250 bis 600° C bei Drücken von > 100kPa umgesetzt werden. Mit der Einstellung des angeführten Massenverhältnisses zwischen MeOH und höheren Alkoholen wird bei der Umsetzung des Alkoholgemisches am Umwandlungskatalysator ein weitgehender Wärmeausgleich zwischen exothermer MeOH-Umwandlung und endothermer Dehydratisierung der höheren Alkohole erreicht, der zu einer annähernd thermoneutralen Reaktion führt. Damit wird die Energie für die Dehydratisierung des Alkohols durch die MeOH-Umwandlung bei gleichzeitiger zusätzlicher Olefinbildung erbracht. Problematisch ist allerdings, dass sich das aus dem eingespeisten EtOH gebildete C₂H₄ mit steigender Verweilzeit im Reaktor zunehmend zu andern weniger wertvollen Produkten wie Olefinen mit mehr als vier Kohlenstoffatomen, Paraffinen, Aromaten und/oder Naphthenen umsetzt. Außerdem besteht das Problem der Temperaturkontrolle. Wenn ein Produkt mit einem definierten Anteil von C₃H₆ und C₂H₄ erzeugt werden soll, dann ergibt sich für die Gesamtreaktion eine adiabate Temperaturerhöhung von 50 bis 150° C. In einem einstufigen Reaktor durchläuft die Reaktion demnach einen breiten Temperaturbereich. Da mit steigender Temperatur die Selektivitäten für C₃H₆ und C₂H₄ zunehmen, andererseits eine für den jeweiligen Katalysator maximal zulässige Temperatur nicht überschritten werden darf, werden die Reaktionsbedingungen umso ungünstiger, je breiter der im Reaktor durchlaufende Temperaturbereich wird, mit der Folge, dass die Ausbeute an Zielprodukten geschmälert wird.

Es ist die Aufgabe der vorliegenden Erfindung, das eingangs beschriebene Verfahren so zu gestalten, dass das Verhältnis von C₂H₄ zu C₃H₆ im Hinblick auf das angestrebte Zielprodukt hinreichend genau einstellbar ist.

Gelöst ist diese Aufgabe dadurch, dass in mehrere Reaktionsstufen des Reaktors EtOH eingespeist wird. Durch diese Maßnahme lässt sich eine bessere Temperatursteuerung der adiabaten Umsetzung des MeOH zu Kohlenwasserstoffen erreichen, da die zur Verdampfung des EtOH benötigte Energie dem bei der exothermen Umsetzung des MeOH gewonnenen Reaktionsprodukt entzogen und dieses dadurch gekühlt wird. Die infolge der Einspeisung von EtOH erzielbare effiziente Temperatursteuerung der bei der adiabaten Umsetzung ablaufenden chemischen Reaktionen führt sowohl zu einer Steigerung der Ausbeute an Zielprodukten als auch zur Einsparung von Apparaten, wie Wärmeübertragern, etc. Die Einspeisung von EtOH in den mehrstufigen Reaktor erfordert zusätzlich lediglich eine mit einer Mengenregelung ausgestattete Aufgabeeinrichtung. Darüber hinaus lässt sich mit EtOH als Zusatzeinspeisung das für das Zielprodukt angestrebtes Verhältnis von C₂H₄ zu C₃H₆ einstellen. Ohne die direkte Rückkühlung der bei der Umsetzung von MeOH erzeugten Reaktionsprodukte wären für die Temperatursteuerung der Umsetzung zusätzliche Apparate, wie außen oder innen liegende Wärmeübertrager mit Kühlmedien sowie Separatoren, externe Kühleinrichtungen etc. und apparatetechnische Komponenten erforderlich. Es ist auch von Vorteil, dass durch die endotherme Umsetzung des EtOH zu C₂H₄ die Reaktionswärme der exothermen Reaktion der Umsetzung des MeOH deutlich reduziert und infolgedessen ein ausgesprochen günstiges Temperaturniveau im Reaktor einstellbar ist.

Bei den in dem Reaktor herrschenden kurzen Verweilzeiten läuft die Dehydratisierung von EtOH zu C₂H₄ ohne erkennbare Weiterreaktion zu unerwünschten Folgeprodukten, wie C₄₊-Olefinen, Paraffinen, Aromaten und/oder Naphthenen ab. Infolge der kurzen Verweilzeiten, die nur durch Zuspeisung von EtOH auf mehrere Reaktionsstufen möglich sind, läuft die Umsetzung vom EtOH und MeOH praktisch unabhängig voneinander ab. Beide vorstehend beschriebenen Effekte ermöglichen eine gezielte Produktverteilung von C₂H₄ und C₃H₆ durch ein entsprechendes Verhältnis der Mengen an dem Reaktor zugespeisten EtOH und MeOH.

Für den Betrieb des Verfahrens ist es von Vorteil, wenn als Festbett-Katalysator ein Zeolith mit Pentasilstruktur, vorzugsweise vom Typ ZSM-5 oder MFI-Z eingesetzt wird.

Vorteilhafterweise werden für die Durchführung des Verfahrens die Umsetzungstemperaturen auf einen Bereich von 360 bis 550° C, vorzugsweise 400 bis 500° C und die Drücke auf einen Bereich von 0,5 bis 5,0 bar[a], vorzugsweise 1,0 bis 3,0 bar[a] eingestellt.

Eine vorzugsweise Ausbildung des erfindungsgemäßen Verfahren ist darin zu sehen, dass für das Herstellen des Zielprodukts 0,1 bis 5,0 kg, vorzugsweise 0.3 bis 3,0 kg, insbesondere 0,2 bis 2,0 kg MeOH pro kg Festbett-Katalysator und Stunde eingesetzt werden und das Gesamt-Massenverhältnis von in den Reaktor eingespeistem EtOH zu dem Reaktor aufgegebenem MeOH (Summe über alle Reaktionsstufen) 0,01 bis 1,0 kg/kg, vorzugsweise 0,02 bis 0,8 kg/kg, insbesondere 0,005 bis 0,5 beträgt.

Die Erfindung wird nachfolgend näher und beispielhaft erläutert:

### Beispiel 1:

Zur Veranschaulichung der Produktverteilung beim Umsetzen von EtOH ohne Zugabe von MeOH werden 50 g eines Zeolith-Katalysators in einen isothermen Festbettreaktor eingefüllt, danach wird auf eine Temperatur von 450° C bei einem Druck von 1 bar[a] aufgeheizt und ein EtOH-Wasser-Gemisch dem Festbettreaktor aufgegeben. Das erzeugte heiße Reaktionsgemisch wird kondensiert und die Gas-Wasser-Phase bzw. etwaige Benzin-Phasen getrennt analysiert. Die Analyse führt zu folgenden Ergebnissen:

| | |
|---|---|
| WHSV (EtOH) | 1 (kg/kg·h) |
| WHSV (H₂O) | 2 (kg/kg·h) |
| X (EtOH) | 100 (%) |
| Y(C₂=) | 97 (mol C/mol C) |
| Y(C₃=) | 1 (mol C/mol C) |
| Y(Olefine) | 99 (mol C/mol C) |
| Y(Paraffine) | 1 (mol C/mol C) |

(WHSV ∼ weight-hourly-space-velocity; X ∼ Umsatz; Y ∼ Ausbeute)

Die Ergebnisse zeigen, dass die Umsetzung mit sehr hoher Ausbeute zu C₂H₄ führt.

### Beispiel 2:

Bei gleichem Verfahrensaufbau und gleicher Verfahrensdurchführung wie in Beispiel 1 wird EtOH in Gegenwart von MeOH umgesetzt. Für das zeitgleiche Umsetzen von EtOH und MeOH lassen sich die Selektivitäten der Umsetzung von EtOH nur durch genaues Trennen und Herausrechnen der in ihrer Selektivitätsverteilung bekannten Umsetzung von MeOH ermitteln. Die Analyse führt zu folgenden Ergebnissen:

| | |
|---|---|
| WHSV (EtOH) | 0,5 (kg/kg·h) |
| WHSV (MeOH) | 0,5 (kg/kg·h) |
| WHSV (H₂O) | 2 (kg/kg·h) |
| X (EtOH) | 100 (%) |
| Y (C₂₌ ex EtOH) | 83 (mol C/mol C) |
| Y (C₃₌) | 10 (mol C/mol C) |
| Andere | 7 (mol C/mol C) |

Eine vergleichende Gegenüberstellung der Analysewerte zeigt, dass die zeitgleiche Umsetzung von EtOH und MeOH weitgehend unabhängig voneinander abläuft.

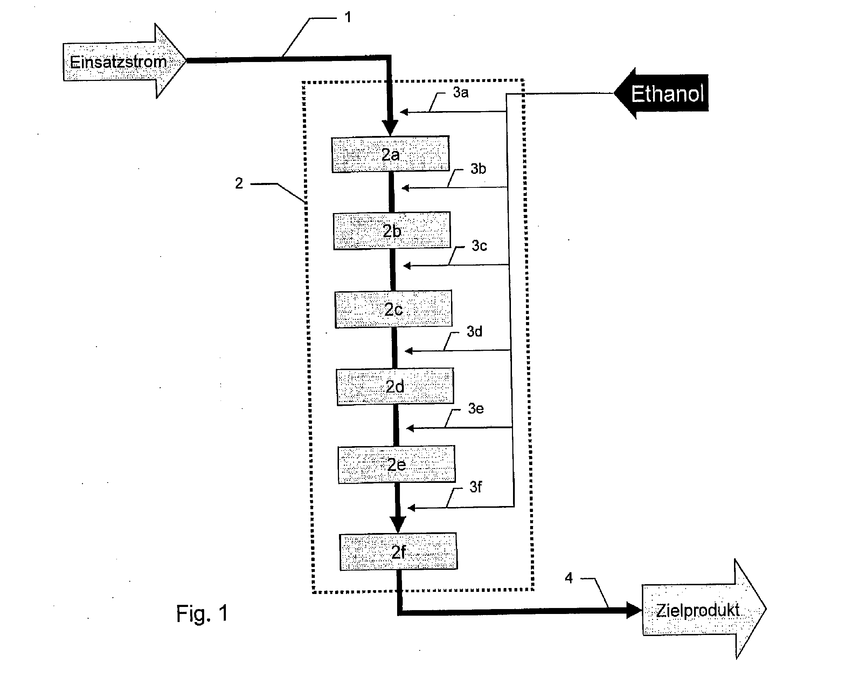

Aus den Ergebnissen der vorstehenden Beispiele 1 und 2 lässt sich folgendes in der Zeichnung in Fig. 1 dargestelltes Ausführungsbeispiel ableiten:
Über die Leitung (1) wird in den Reaktor (2), in dem sechs senkrecht nacheinander geschaltete Reaktionsstufen (2a, 2b, 2c, 2d, 2e, 2f) eingebaut sind, über Kopf ein gasförmiger Einsatzstrom, im wesentlichen bestehend aus 40 Gew. % MeOH und DME, 25 Gew. % Wasser und 35 Gew. % Kohlenwasserstoffen, eingeleitet und mit einer Temperatur von 460° C bei einem Druck von 2,3 bar[a] der ersten Reaktionsstufe (2a) zugeführt. In den Einsatzstrom und in die Zwischenproduktströme der Reaktionsstufen (2b, 2c, 2d, 2e, 2f) wird über die Leitungen (3a, 3b, 3c, 3d, 3e, 3f) flüssiges EtOH in einer Gesamtmenge von EtOH_{gesamt} zu MeOH_{gesamt} von 1 : 10 eingespeist. Am Austritt des Reaktors (2) wird über Leitung (4) ein Produktstrom (Zielprodukt) abgezogen und zur Aufarbeitung und Trennung der Komponenten ausgeleitet.

Wird in den Einsatzstrom sowie in die Produktströme zwischen den Reaktionsstufen kein flüssiges EtOH eingeleitet, so ergeben sich folgende Umsätze (Referenzwerte):
C₃₌ ∼ 100 %; C₂₌ ∼ 100 %; C₂₌ / C₂₌ + C₃₌ ∼ 2,4 %.
   Durch den Zusatz von flüssigem EtOH verbessern sich die Umsätze wie folgt:
C₃₌ ∼ 103 %; C₂₌ ∼ 490 %; C₃₌ / C₂₌ + C₃₌ ∼ 10,9 %.
   Die zusätzlich Bildung von C₃H₆ ergibt sich teilweise aus dem besseren Temperaturprofil unter den Bedingungen des Zusatzes von EtOH, wodurch die Eintrittstemperaturen pro Reaktionsstufe um 2 bis 5° C angehoben werden, ohne dass die Maximaltemperatur am Ende jeder Reaktionsstufe überschritten wird.

Mit steigender Zahl an Reaktionsstufen nimmt der positive Effekt der hochselektiven Umsetzung von EtOH zu C₂H₄ ab, d.h. je weiter hinten in dem mit mehreren Reaktionsstufen ausgerüsteten Reaktor die Einspeisung von EtOH erfolgt, desto besser ist die zielgerichtete Ausbeute der eingespeisten Menge an EtOH.

Das erfindungsgemäße Verfahren ermöglicht somit eine gezielte Einstellung der notwendigen Produktverteilung von C₂H₄ und C₃H₆ für die Herstellung von Polypropylen-Copolymeren

Einen weiterer mit der Erfindung erzielte Vorteil besteht darin, dass für die Durchführung des Verfahrens auf den Einsatz von reinem EtOH (>99 %) verzichtet und technisches EtOH (94 %, Rest Wasser) benutzt werden kann, da auf Grund der positiven Wirkung der mehrstufigen Einspeisung von EtOH der Wasseranteil nicht nur nicht stört, sondern auch für den Ablauf der Gesamtreaktionen hilfreich ist; beispielweise werden Verkokungserscheinungen stärker unterdrückt und die adiabate Temperaturerhöhung gesenkt.

Der Effekt der separat ablaufenden Umsetzung des EtOH bei gleichzeitig hohen Ausbeuten an C₂H₄ und C₃H₆ kann praktisch nur durch die mehrstufige Einspeisung des EtOH realisiert werden, da so zumindest der in die letzte Reaktionsstufe eingespeiste EtOH -Teilstrom mit voller Ausbeute zu den Zielprodukten , insbesondere C₂H₄, abreagiert. Wird jedoch das EtOH komplett in eine Reaktionsstufe dosiert so laufen eine steigende Menge unerwünschter Folgereaktionen aus dem zu Beginn gebildeten C₂H₄ ab.

## Patentansprüche

1. Verfahren zum Herstellen eines C₃H₆ und C₂H₄ enthaltenden Produkts durch zeitgleiches Umsetzen von MeOH und EtOH in einem mehrere Reaktionsstufen enthaltenden adiabaten, sequentiell betriebenen Reaktor, wobei jede Reaktionsstufe mit einem Festbett aus formselektiven Katalysator belegt ist, indem ein gasförmiger MeOH, DME, H₂O und ggf. einen oder mehrere C₂-, C₃-, C₄-, C₅-,C₆-, C₇-, C₈- Olefine und -Paraffine enthaltender Einsatzstrom mit Temperaturen von 300 bis 600° C bei Drücken von 0,1 bis 20 bar[a] wenigstens der ersten Reaktionsstufe des Reaktors aufgegeben wird, **dadurch gekennzeichnet, dass** in mehrere Reaktionsstufen des Reaktors EtOH eingespeist wird.

2. Verfahren nach Anspruch1, **dadurch gekennzeichnet, dass** der Festbett-Katalysator aus Zeolith vom Pentasiltyp, vorzugsweise des Typs ZSM-5, besteht.

3. Verfahren nach einem der Ansprüche 1 und 2, **dadurch gekennzeichnet, dass** die Umsetzung bei Temperaturen von 360 bis 550° C, vorzugsweise 400 bis 500° C durchgeführt wird.

4. Verfahren nach einem der Ansprüche 1 bis 3, **dadurch gekennzeichnet, dass** die Umsetzung bei Drücken von 0.5 bis 5,0 bar[a], vorzugsweise 1,0 bis 3,0 bar[a] durchgeführt wird.

5. Verfahren nach einem der Ansprüche 1 bis 4, **dadurch gekennzeichnet, dass** 0,1 bis 5,0 kg, vorzugsweise 0,3 bis 3,0 kg, insbesondere 0,2 bis 2,0 kg MeOH pro kg Festbett-Katalysator und Stunde eingesetzt werden und das Gesamt-Massenverhältnis von eingespeistem EtOH zu aufgegebenem MeOH 0,01 bis 1,0 kg/kg, vorzugsweise 0,02 bis 0,8 kg/kg, insbesondere 0,005 bis 0,5 kg/kg beträgt.

## Claims

1. A process for producing a product containing C₃H₆ and C₂H₄ by simultaneous conversion of MeOH and EtOH in an adiabatic, sequentially operated reactor containing a plurality of reaction stages, wherein each reaction stage is occupied with a fixed bed of a form-selective catalyst, in that a gaseous feed stream containing MeOH, DME, H₂O and possibly one or more C₂-, C₃-, C₄-, C₅-, C₆-, C₇-, C₈-olefins and -paraffins is charged at least to the first reaction stage of the reactor with temperatures of 300 to 600°C at pressures of 0.1 to 20 bar[a], **characterized in that** EtOH is fed into more than one of the reaction stages of the reactor.

2. The process according to claim 1, **characterized in that** the fixed-bed catalyst consists of zeolite of the pentasil type, preferably of the type ZSM-5.

3. The process according to any of claims 1 and 2, **characterized in that** the reaction is carried out at temperatures of 360 to 550°C, preferably 400 to 500°C.

4. The process according to any of claims 1 to 3, **characterized in that** the reaction is carried out at pressures of 0.5 to 5.0 bar[a], preferably 1.0 to 3.0 bar[a].

5. The process according to any of claims 1 to 4, **characterized in that** 0.1 to 5.0 kg, preferably 0.3 to 3.0 kg, in particular 0.2 to 2.0 kg MeOH per kg of fixed-bed catalyst and hour are used and the total mass ratio of fed EtOH to charged MeOH is 0.01 to 1.0 kg/kg, preferably 0.02 to 0.8 kg/kg, in particular 0.005 to 0.5 kg/kg.

## Revendications

1. Procédé de production d'un produit contenant du C₃H₆ et du C₂H₄ en transformant en même temps du MeOH et de l'EtOH dans un réacteur comportant plusieurs étages de réaction, adiabatique et séquentiel, chaque étage de réaction étant occupé par un lit fixe en un catalyseur à sélection de forme, en chargeant un courant de charge gazeux contenant du MeOH, du DME, de l'H₂O et, le cas échéant, une ou plusieurs oléfines et paraffines en C₂, C₃, C₉, C₅, C₆, C₇, C₈ à des températures de 300 à 600°C sous des pressions de 0,1 à 20 bar[a] dans au moins le premier étage de réaction du réacteur, **caractérisé en ce qu'**on injecte de l'EtOH dans plusieurs étages de réaction du réacteur.

2. Procédé suivant la revendication 1, **caractérisé en ce que** le catalyseur en lit fixe est en zéolithe de type pentasile, de préférence du type ZSM-5.

3. Procédé suivant l'une des revendications 1 et 2, **caractérisé en ce qu'**on effectue la réaction à des températures de 360 à 550°C, de préférence de 400 à 500°C.

4. Procédé suivant l'une des revendications 1 à 3, **caractérisé en ce qu'**on effectue la réaction sous des pressions de 0,5 à 5,0 bar[a], de préférence de 1,0 à 3,0 bar[a].

5. Procédé suivant l'une des revendications 1 à 4, **caractérisé en ce qu'**on engage de 0,1 à 5,0 kg, de préférence de 0,3 à 3,0 kg, notamment de 0,2 à 2,0 kg de MeOH par kg de catalyseur en lit fixe et par heure, et **en ce que** le rapport massique total de l'EtOH injecté au MeOH chargé va de 0,01 à 1,0 kg/kg, de préférence de 0,02 à 0,8 kg/kg, notamment de 0,005 à 0,5 kg/kg.
